# EUROPEAN PATENT APPLICATION

(11) **EP 2 138 586 A1**
(43) Date of publication of application: **30.12.2009**
(21) Application number: 08011388.9
(22) Date of filing: 24.06.2008
(51) Int. Cl.: C12P 19/04, C12P 19/12

(54) **Process for the regioselective preparation of disaccharides or oligosaccharides**

(71) Applicant: Cognis IP Management GmbH, 40589 Düsseldorf (DE); Universidad Complutense De Madrid, 28040 Madrid (ES)
(72) Inventor: Bigorra Llosas, Joaquin, 08201 Sabadell (ES); Raya, Javier, 08970 Sant Joan Despi (Barcelona) (ES); Fabry, Bernd, 41352 Korschenbroich (DE); Valls, Ramon, 08022 Barcelona (ES); Perez, Maria, 28022 Madrid (ES); Hernaiz, Maria Jose, 28010 Madrid (ES); Sinisterra, Jose-Vicent, 28013 Madrid (ES)

(57) **Abstract**

Suggested is a process for the regio-selective manufacture of disaccharides or oligosaccharides by transglycosilation or hydrolysis of a carbohydrate donator molecule [G-R] and a glycosyl acceptor molecule [A] in the presence of a glycosidase in the presence of at least one dialkyl amide.

## Description

### Field of the invention

The present invention is related to the area of carbohydrates and refers to a new process for the regio-selective manufacture of di- and oligosaccharides.

### Background of the invention

A recent expansion of our understanding of the biological role and clinical utility of oligosaccharides has created a need for new synthetic methodologies for inexpensively producing large quantities of these compounds with high regio-selectivity. Particularly, β-di- and - oligosaccharides like e.g. β-1,6-galactosides or 1,4 β- galactosides represent important classes of carbohydrates for which many new bioactivities have recently been characterized and for which new synthetic routes are needed. Conventional chemical and existing enzymatic methodologies for synthesizing di- or oligosaccharides with high regio-selectivity are inadequate for commercial application, since they are complex, involve many steps and include undesired side reactions.

When enzymatic methodologies are available for synthesizing di- and oligosaccharides, they are often preferred over organic processes. As compared to chemical methodologies, many enzymatic routes are characterized by their relative. For example enzymatic transglycosilation, that means the transfer of a glycosidically bounded sugar to the hydroxyl moiety of another molecule in the presence of a bio-catalyst, usually leads to a mixture of isomers.. For example US 5,876,981 (Scripps) discloses β-galactosides which were synthesized using a transglycosylation reaction catalyzed by β-galactosidase. The reaction employs a carbohydrate donor having a glycosidic leaving group attached to its anomeric carbon and an oxo group attached to the C-6 carbon. The method is carried out in aqueous solution without organic solvents.

US 5,955,324 (Takara) discloses a process for producing a carbohydrate or a glycoconjugate by a remodeling reaction in the presence of a glycosidase which performs the reaction in an aqueous medium containing acetone and/or dioxane.

Therefore, the problem underlying the present invention has been to improve velocity of the enzymatic hydrolysis of substituted carbohydrates and to improve regio-selectivity in transglycosylation towards the production of one of the isomers. It has been particular desirous to obtain one of the isomers in ratios by weight of more than 90 % and preferably more than 95 % calculated on the isomers.

### Detailed description of the invention

The present invention refers to a process for the regio-selective manufacture of disaccharides or oligosaccharides by transglycosilation or hydrolysis of a carbohydrate donator molecule [G-R] and a glycosyl acceptor molecule [A] in the presence of a glycosidase in the presence of at least one dialkyl amide.

Another object of the invention is directed to a process for the production of carbohydrates, which is characterised in that carbohydrate donor molecules [G-R] are subjected to hydrolysis in the presence of an enzyme capable to hydrolyse the bond between the saccharide moiety [G] and the leaving group [R], in the presence of at least one dialkyl amide.

Surprisingly it has been observed that adding dialkyl amides to the reaction mixture, hydrolysis is usually accelerated and the transglycosylation leads to the production of one of the isomers in high yield in high yields and ratios by weight of more than 90 %, and preferably more than 95 % - calculated on the isomers.

### Carbohydrate donator molecules [G-R]

As explained above, the process employs a suitable enzyme, β-glycosidase, β-galactosidase, β-fucosidase, β-N-acethyl-glucosaminidase, preferably a β-galactosidase for catalyzing a transglycosylation reaction between a first galactosidase substrate [G-R] and a second galactosidase substrate [A] for producing the transglycosylation product. The first substrate is a carbohydrate donor [G-R] represented by the following formula (**I**),

[G]-[R] (I)

in which [G] represents a monosaccharide, disaccharide or oligosaccharide moiety having an anomeric carbon and [R] stands for a leaving group attached to said anomeric carbon. Typical examples for suitable saccharides [G] are monosaccharides, preferably having 4 to 6 carbon atoms like
o tetroses, like e.g. erythrose, threose or erythrulose;
o Pentoses, like arabinose, lyxose, ribose, deoxyribose, xylose, ribulose or xylulose;
o hexoses, like e.g. allose, altrose, galactose, glucose, gulose, idose, mannose, talose, fructose, psicose, sorbose or tagatose;

Suitable disaccharides are sucrose, lactose, maltose, trehalose and cellobiose, an example for a suitable oligosaccharide is inulin.

The oxo group may be in equilibrium between an aldehyde form and acetal form. Typical examples for suitable leaving groups [R] are fluor, thio-glicosides, nitrophenyl groups, or a non activated donor, for example hydrogen, a monosacharide or a polysacharide. Particular useful as a leaving group are nitrophenyl groups according to formula (III)

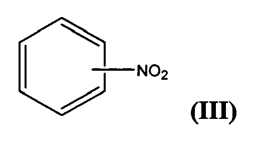

The ortho and para isomers are the preferred leaving groups.

### Glycosyl acceptor molecules [A]

The second substrate is a glycosyl acceptor. Suitable acceptor molecules [A] are monosaccharides, disaccharides, oligosaccharides or fatty alcahols showing a similar or different structure compared to group [G] in formula (I) explaining the nature of the donator molecules. These molecules may be substituted by additional groups which are different from the leaving groups [R] of formula (I). For example, suitable substituents are ester or amide groups. Preferred carbohydrate acceptor molecules include N-acetyl-D glucosamine and N-acetyl-D glucosamine glycoside.

### Enzymes

According to the process of the present invention basically all enzymes are suitable being capable to cause the transfer of a saccharide group from a donator to an acceptor molecule. The preferred enzymes are β-galactosidases which are obtainable for example from *Escherichia coli, Bacillus circulans, Aspergillus arycae* or *Penicillium simplicissium.* Typically the enzymes are used in quantities of about 100 to 200 U.

### Dialkyl amides

Dialkyl amides which are useful as regio-selective solvents according to the present invention are water soluble and follow formula (IV)

R¹CO-NR²R³ (IV)

in which R¹CO stands for a linear or branched, saturated or unsaturated, optionally hydroxy-substituted radical having 1 to 21 carbon atoms, and R² and R³ independently from each other represent alkyl radicals having 1 to 6 carbon atoms. Typical examples of suitable dialkyl amides are diethylamides, dipropyl amides, dibutylamides, dipentylamides, methylethyl amides and preferably dimethyl amides based on saturated or unsaturated fatty acids like capric acid, caprylic acid, caprinic acid, lauric acid, myrystic acid, palmitic acid, palmoleic acid, stearic acid, cetearic acid, oleic acid, elaidinic acid, linolic acid, linoleinic acid, conjugated linoleic acid, ricinoleic acid, 12-hydroxystearic acid, gadoleinic acid, arachidonic acid, erucic acid, behenic acid and their mixtures. Also useful are amides based on short chain hydroxy carboxylic acids, like e.g. lactic acid, glycolic acid or citric acid. The preferred amides are dimethyl-amides based on C₆-C₁₀ fatty acid, C₈-C₁₀ fatty acid, lactic acid and their mixtures. In order to achieve a sufficient acceleration of the hydrolysis or transglycosidation and to shift the selectivity towards the desired β-1,6 isomers it has been found advantageous to use the dialkyl amides in concentrations of about 0.1 M to about 5.0 M, preferably 0.2 M to 2.0 M and more preferably 0.5 M to 1.0 M - calculated on the total reaction mixture.

### Transglycosylation process

In a preferred mode, the transglycosylation reaction is catalyzed with a molar excess of the second galactosidase substrate [A] as compared to the first galactosidase substrate [G-R]. Typically, from 1 to 10, preferably 3 to 5 molar excess can be employed. Usually, the reaction is conducted in a mixture of an aqueous buffer and the organic solvent. Preferably the weight ratio between both solvents is about 50:50 to about 90:10, and preferably about 80:20. The transglycosylation is carried out at ambient temperatures which are mostly defined by the optimal working temperature of the enzyme, which lies between about 20 and about 40 °C.

### Industrial application

The pure di- and oligosaccharides isomers are useful for quite a number of applications. Examples may be:
- food industry, especially for sweetening agents and lactose-free products;
- cosmetic industry, especially for skin care ingredients like glycolipids, ceramide analogues;
- pharmaceutical industry, especially for the synthesis of actives for fighting coagulation, cancer, VHS, inflammation, virus and bacterial infections.

### Examples

### Examples 1 and 2

### Transglycosilation of p-nitrophenyl-β-D-galactopyranoside and N-acetylglucosamine using lactic acid dimethylamide as a solvent

The transglycosilation of p-nitrophenyl-β-D-galactopyranoside and N-acetylglucosamine conducted in the presence of the ß-galactosidase *B. circulans* has been reported to yield dominantly the Gal-β-1-4-GlcNAc isomer, while the Gal-β-1-6-GlcNAc isomer is obtained in poor yields as a by-product. The reaction is shown in the following figure:

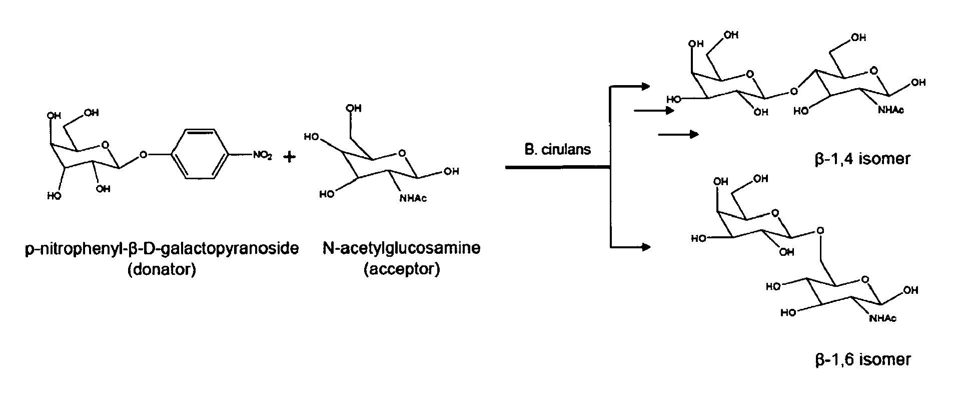

In the following the influence of the solvent on the behaviour of the enzyme has been studied by carrying out the reaction without solvent (buffer only) and adding lactic acid dimethyl amide (LDMA) or caprylic dimethyl amide (CDMA) at concentrations of 0.5 M and 2 M. As buffer a mixture of citrate and phosphate (50 mM, pH 5) was used. The reaction was conducted by adding 155 U of *B. circulans* at a temperature of about 30 °C. Donator and acceptor were brought in at a concentration of 0.17 M and 0.85 M respectively. In order to follow the reaction samples of 50 µL were taken and heated to 100°C within 5 minutes, the solvent was removed under vacuum, and finally the sample was once again diluted with the same volume of the solvent. The disappearance of the donator was followed by HPLC (Agilent Technologies 1200) equipped with an UV detector and a column (Mediterraneasea 18 16 cm*0.46 5 µm, Teknokroma). The results are reflected in Table 1:

**Table 1**

| Decrease of donator during the time of reaction Distribution of reaction products depending on the solvent; ratio by weight buffer:DMA = 80:20 | | | | | |
|---|---|---|---|---|---|
| **Solvent** | **log P** | **Donor (pN02-β-Gal)** | **Gal** | **B (1-4) Isomer [%]** | **B (1-6) isomer [%]** |
| Buffer (100%) | 0 | - | - | 83 | 17 |
| LDMA | -0.69 | 5 | - | - | 95 |
| CDMA | 1.42 | 10 | 51 | - | 90 |

In order to determine whether not only hydrolysis, but also transglycosilation has taken place the reaction products were analysed by HPLC equipped with light scattering (JASCO HPLC) or refraction index (WATERS HPLC) detector and a column (Carbotracer-NH2 25*0.47 5 µm Teknochroma). As one can see, adding the lactic or C-6 dimethylamide to the reaction mixture leads to significant change in regio-selectivity.

## Claims

1. A process for the regio-selective manufacture of disaccharides or oligosaccharides by transglycosilation or hydrolysis of a carbohydrate donator molecule [G-R] and a glycosyl acceptor molecule [A] in the presence of a glycosidase in the presence of at least one dialkyl amide.

2. A process according to Claim 1, **characterised in that** said disaccharides or oligosaccharides are β-1,6-disaccharides or β-1,6-oligosaccharides.

3. A process according to Claims 1 and/or 2, **characterised in that** said carbohydrate donor molecules [D] follow formula (I)
[G]-[R] (I)
in which [G] represents a monosaccharide, disaccharide or oligosaccharide moiety having an anomeric carbon and [R] stands for a leaving group attached to said anomeric carbon.

4. A process according to any of the preceding Claims 1 to 3, **characterised in that** said carbohydrate donor molecules [G-R] include as the leaving group [R] hydrogen, fluor, a nitrophenyl group, a monosaccharide, a polysaccharide or a thioglycoside.

5. A process according to any of the preceding Claims 1 to 4, **characterised in that** said glycosyl acceptor molecules [A] represent monosaccharides, disaccharides or oligosaccharides, or a fatty alcohol, optionally substituted by an ester or amide group.

6. A process according to any of the preceding Claims 1 to 5, **characterised in that** said glycosyl acceptor molecule [A] represent N-acetyl-D glucosamine or N-acetyl-D glucosamine glycoside.

7. A process according to any of the preceding Claims 1 to 6, **characterised in that** said enzyme is a β-galactosidase.

8. A process according to any of the preceding Claims 1 to 7, **characterised in that** said dialkyl amides follow formula (IV)
R¹CO-NR²R³ (IV)
in which R¹CO stands for a linear or branched, saturated or unsaturated, optionally hydroxysubstituted radical having 1 to 21 carbon atoms, and R² and R³ independently from each other represent alkyl radicals having 1 to 6 carbon atoms.

9. A process according to Claim 7, **characterised in that** said dialkyl amides are dimethylamides based on C₆-C₁₀ fatty acid, C₈-C₁₀ fatty acid, lactic acid or their mixtures.

10. A process for the production of carbohydrates, **characterised in that** carbohydrate donor molecules [G-R] according to any of the preceding Claims 1 to 9 are subjected to hydrolysis in the presence of an enzyme capable to hydrolyse the bond between the saccharide moiety [G] and the leaving group [R], and at least one dialkyl amide.
